(19) Europäisches Patentamt
European Patent Office
Office européen des brevets

(11) Numéro de publication: 0 081 408
B1

(12) # FASCICULE DE BREVET EUROPÉEN

(45) Date de publication du fascicule du brevet: **22.07.87**

(51) Int. Cl.⁴: **C 08 F 8/32**, C 07 K 1/04, C 07 K 1/06, C 07 K 1/10

(21) Numéro de dépôt: **82402109.1**

(22) Date de dépôt: **19.11.82**

(54) **Nouveaux copolymères acryliques à fonctionnalisation aminée et leur préparation.**

(30) Priorité: **19.11.81 GB 8134861**

(43) Date de publication de la demande: **15.06.83 Bulletin 83/24**

(45) Mention de la délivrance du brevet: **22.07.87 Bulletin 87/30**

(84) Etats contractants désignés: **AT BE CH DE FR GB IT LI LU NL SE**

(56) Documents cité:
**FR-A-2 157 628**
**FR-A-2 205 538**
**FR-A-2 275 482**

(73) Titulaire: **Société d'Expansion Scientifique EXPANSIA Société Anonyme, 51/53 rue du Docteur Blanche, F-75016 Paris (FR)**

(72) Inventeur: **Aspisi, Christian, Les Grands Vallons Chemin du Breuil, F-13150 Boulbon (FR)**
Inventeur: **Calas, Bernard, 9, rue de la Traversière, F-34980 St.- Gely du Fesc (FR)**
Inventeur: **Daunis, Jacques, 38 Bld. Ernest Renan, F-34000 Montpellier (FR)**
Inventeur: **Follet, Michel, 1 Av. de la Libération, F-30390 Aramon (FR)**
Inventeur: **Jacquier, Robert, 445 Av. Valéry Larbaud, F-34000 Montpellier (FR)**
Inventeur: **Parello, Joseph, 26 rue Haguenot, F-34000 Montpellier (FR)**

(74) Mandataire: **Lachassagne, Jacques, Boite Postale 07, F-78430 Louveciennes (FR)**

**EP 0 081 408 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

## Description

La présente invention concerne de nouveaux copolymères acryliques, leur préparation ainsi que leur emploi dans la synthèse peptidique en phase solide.

Les nouveaux copolymères selon l'invention dérivent de ceux décrits par la demanderesse dans sa demande de brevet publiée EP-A-0 079 842 par une fonctionnalisation à l'aide de l'éthylènediamine. Les copolymères de la demande précédente - que l'on désigne par A ou

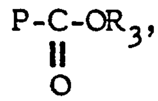

avec $R_3$ = H ou $-CH_3$ - étaient obtenus par copolymérisation des trois monomères suivants:

I - Un monomère défini comme étant la maille du support et qui consiste en une N-acrylylpolyméthylèneimine de formule:

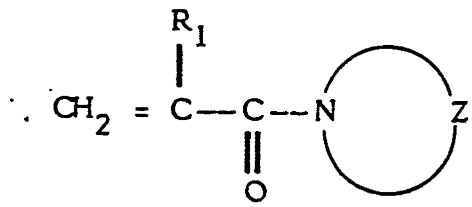

avec $R_1$ = H ou $-CH_3$

$Z = -(CH_2)_{n_1}-$ avec $n_1$ = 4, 5 ou 6

ou

$-(CH_2)_2-X-(CH_2)_2-$ avec X = O ou $\overset{|}{\underset{|}{N}}-CH_3$

ou un N-acrylyldialkylamide, de formule:

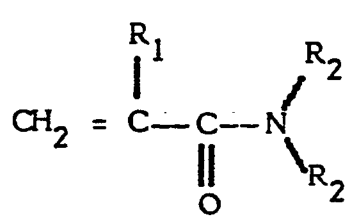 2

avec $R_1$ ayant la signification précédente et $R_2$ = $-CH_3$ ou $-C_2H_5$

II - Un monomère défini comme étant l'agent de réticulation et qui consiste en un N,N'-bisacrylyldiaminoalcane de formule:

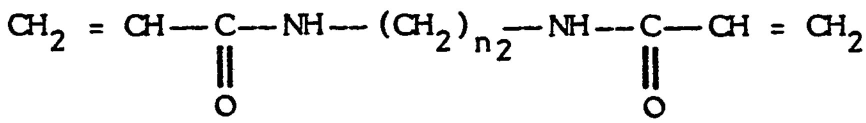

3

avec $n_2$ = 1 ou 2

III - Par gramme de support sec, 0,2 à 5 mmol d'un monomère défini comme étant l'agent de fonctionnalisation et qui consiste en un N-acrylylaminoacide ou ester, de formule:

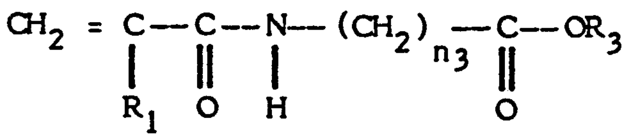

4

avec $R_1$ et $R_3$ = H ou-$CH_3$ et $n_3$ = 1, 2, 3 ou 5

ou un N-acrylylaminoacide (ou ester) asymétrique de série L, de formule:

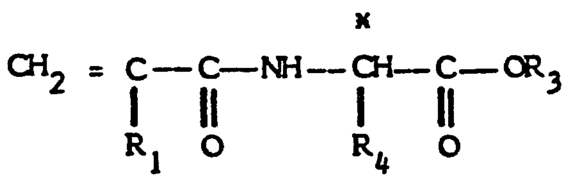

5

$R_1$ et $R_3$ ayant la signification précédente et

$$R_4 = -CH_3$$

$$-CH = (CH_3)_2$$

$$-CH_2-CH = (CH_3)_2$$

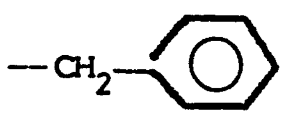

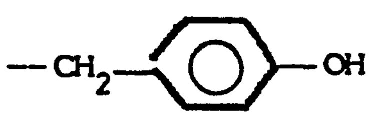

$$-CH_2-CH_2-S-CH_3$$

$$-(CH_2)_4-NH_2$$

Ou encore la N-acrylyl (L) proline, ou son ester méthylique, de formule:

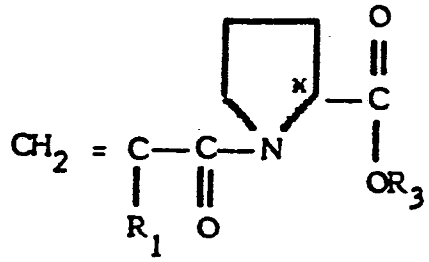

6

avec $R_1$ et $R_3$ ayant la signification précédente.

La fonctionnalisation pour obtenir les copolymères selon l'invention s'effectue en créant un bras par amidification avec l'éthylènediamine, pour donner B:

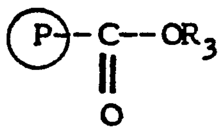

A

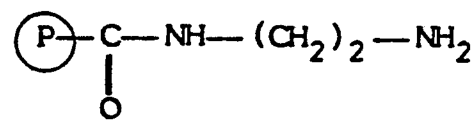

B

Cette amidification peut être réalisée par l'une des voies suivantes:

a) à partir de la fonction acide (A, $R_3$ = H), par couplage avec l'éthylène diamine au moyen de l'un des réactifs couramment utilisé dans la littérature: dicyclohexylcarbodiimide (DCC) en présence d'un agent d'activation comme l'hydroxy-1 benzotriazole (HOBt), ou le benzotriazol-1-yl-oxy-tris (diméthylamino) phosphonium hexafluoro phosphate (BOP);

b) à partir de la fonction ester (A, $R_3$ = $CH_3$), par agitation de la résine à température ambiante avec un excès d'éthylènediamine anhydre.

Avec cette dernière méthode, une étude systématique des conditions expérimentales a permis de limiter les réactions secondaires de pontage et de conserver 90 % du taux de fonctionnalisation initial.

La méthode de synthèse peptidique en phase solide, qui s'est développée depuis 1962 en utilisant principalement des supports polystyréniques (résine de MERRIFIELD, résine à la benzhydrylamine, résine alcoxybenzylique, résine PAM,...) est bien connue et a fait l'objet de nombreuses mises au point (par exemple ERICKSON & MERRIFIELD, in NEURATH et HILL, The Proteins, Vol. 2, Academic Press, 1976, p. 255; BIRR, Aspects of the Merrifield peptide synthesis, Springer 1978; BARANY et MERRIFIELD, in GROSS et MEIENHOFER, The peptides: analysis, synthesis, biology, Vol. 2, Part A, Academic Press, 1980, p.1). La résine, convenablement fonctionnalisée, est estérifiée ou amidifiée par l'aminoacide N-protégé occupant dans le peptide à synthétiser la position C-terminale. Après déprotection de la fonction amine, le couplage est effectué, en utilisant l'une des nombreuses méthodes décrites dans la littérature, avec l'aminoacide N-protégé occupant l'avant-dernière position dans la séquence à synthétiser. Ces opérations sont répétées jusqu'à obtention sur la résine de l'enchaînement peptidique désiré. Dans la dernière étape, le peptide est clivé de son support.

Cette méthode présente des avantages certains par rapport à la synthèse en solution: en particulier, simplicité et rapidité du processus (il n'est plus nécessaire d'isoler et de purifier le peptide après chaque couplage), élimination aisée des réactifs en excès et des impuretés par filtration et lavage du support, absence ou forte diminution de la racémisation. Une contrainte concerne toutefois l'obligation de parvenir, pour chaque couplage, à un rendement pratiquement quantitatif; chaque réaction incomplète conduirait en effet à une accumulation de chaînes peptidiques tronquées qui contamineraient le produit final. D'où la nécessité d'utiliser des excès importants en réactifs et de répéter chaque opération de couplage jusqu'à ce qu'un test analytique approprié (à la ninhydrine par exemple) indique la disparition sur la résine de toute fonction $NH_2$ libre. De telles conditions expérimentales deviennent cependant dissuasives lorsque la synthèse implique l'emploi d'aminoacides non naturels, éventuellement rares et coûteux.

De façon à tirer profit des avantages associés aux réactions intervenant en phase homogène, il a été proposé d'utiliser des polymères solubles (cf. MUTTER et BAYER, in GROSS et MEINHOFER, The peptides: analysis, synthesis, biology, Vol. 2, Part A, Academic Press, 1980, p.285), et en particulier le polyéthylèneglycol. Cette technique est cependant restée d'une utilisation limitée, vrai semblablement en raison des problèmes expérimentaux posés par.:

- la diminution de solubilité pouvant accompagner l'allongement de la chaîne peptidique,

- l'isolement du support après chaque couplage, qui nécessite une ultrafiltration (MUTTER, HAGENMAIER et BAYER, Angew. Chem. Int. Ed., 1971, 10, 811; BAYER, GATFIELD, MUTTER et MUTTER, Tetrahedron, 1978, 34, 1829), ou une cristallisation par addition d'éther, après éventuelle élimination du solvant (BAYER et MUTTER,

Chem. Ber., 1974, 107, 1344; MUTTER, UHMANN et BAYER, Ann. Chem., 1975, p. 901 CORRING et JUNG, Ann. Chem., 1975, p. 1765 et 1776)

- le clivage du peptide de son support (TJOENG, TONG et HODGES, J. Org. Chem., 1978, 43, 4190; TJOENG et HODGES, Tetrahedron Letters, 1979, p. 1273 ; MUTTER, BAYER et GATFIELD, J. Org. Chem., 1980, 45, 5364).

Les mêmes difficultés interviennent dans le cas des supports solubles à base de polystyrène faiblement ou non réticulé, qui impliquent une filtration de gel (BIRR, in Peptides "1972", North Holland Publ., 1973, p. 72; ANDRETTA et RINK, Helv. Chim. Acta, 1973, 56, 1205) ou une précipitation par l'alcool (NARITA, Bull. Chem. Soc. Japan, 1978, 51, 1477).

Enfin la technique alternée phase liquide-phase solide (FRANK, MEYER e t HAGENMAIER, Chem. Zeit., 1977, 101, 188) s'est également peu développée.

Plus récemment, la supériorité des supports polyacryliques comme le PAP de WALTER (J. Amer. Chem. Soc., 1979, 101, 5383) et la résine de SHEPPARD (JCS Perkin I, 1981, p. 529 et 538), qui possèdent la propriété de gonfler dans une bien plus large gamme de solvants que les polymères styréniques, a été mise en évidence (ATHERTON, GAIT, SHEPPARD et WILLIAMS, Bioorg. Chem. 1979, 8, 774). Il y a, en particulier: un clivage plus facile en fin de synthése, entre le polypeptide et son support; une meilleure compatibilité entre les propriétés de solvatation du support et de la partie peptidique en train de croître, ce qui est de nature à limiter les troncatures et les couplages aberrants. L'influence favorable de la solvatation du système résine-peptide sur le rendement des couplages a particulièrement été mise en évidence (ATHERTON, WOOLNEY et SHEPPARD, Chem. Corrm., 1980, p. 970). Toutefois, les corrmentaires que nous avons déjà formulés dans le cas des résines polystyréniques, en ce qui concerne l'emploi d'un large excès de réactifs (de l'ordre de 2,5 fois) et la nécessité de répéter généralement à plusieurs reprises chaque opération de couplage, restent toujours valables. A cet égard, il est significatif que des synthèses récentes de polypeptides à longue chaîne utilisent encore la méthode en solution (par exemple KENNER, RAKAGE et SHEPPARD, Tetrahedron, 1979, 35, 2767; NAGARAJ et BALARAN, Tetrahedron, 1981, 37, 1263; FUJII et YAJIMA, JCS Perkin I 1981, p.789, 787 et 804.

Afin de pallier les différences de solvatation déjà évoquées plus haut, entre la matrice et la chaîne polypeptidique en train de croître, un nouveau gel polyacrylamide a été récemment proposé (R. EPTON et A. WILLIAMS Inst. J. Biol. Macromol. 1981, 3, 334). Sur ce support, l'encombrement stérique est tel que ce type de résine n'a été utilisé que pour la synthèse de peptides courts. Enfin la déprotection finale nécessite un temps très long de traitement au trifluorure de bore, (plusieurs jours).

Comme on le verra ci-après, les supports faisant l'objet de la présente invention permettent une simplification et une amélioration notables des conditions de la synthèse peptidique; ils peuvent être utilisés tels quels si les peptides synthétisés n'ont pas besoin d'être séparés de leur support. Si les peptides doivent être séparés, il est, par contre, nécessaire d'apporter une modification de la résine selon l'invention en introduisant un système d'ancrage réversible. A titre d'exemple non limitatif, il est possible d'utiliser un réactif du type C (MITCHELL, ERICKSON, RYABTSEV, HODGES et MERRIFIELD, J. Amer. Chem. Soc., 1976; STAHL, WALTER et SMITH, J. Amer. Chem. Soc., 1979, 101, 5383; ATHERTON, LOGAN et SHEPPARD, JCS Perkin I, 1981, p. 538; ATHERTON, HUBSCHER, SHEPPARD et WOOLEY, 2. Physiol. Chem. 1981, 362, 833).

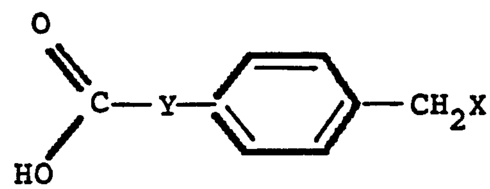

C

dans lequel X = Cl, Br ou OH

Y est un groupe $-CH_2-O-$ ou un groupe $-CH_2$

La réaction entre B et C pour donner D:

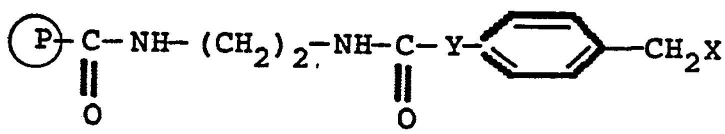

D

peut s'effectuer pratiquement quantitativement au moyen de l'un des agents de couiplage ou catalyseur précédemment cité, ou par action de B sur un ester activé (préparé avec le trichloro-2,4,5 phénol le pentachlorophénol...) de C, en présence ou non d'un catalyseur (HOBT, BOP).

Un autre système d'ancrage reversible a été réalisé en utilisant un réactif du type E ($R_5$ = H ou $CH_3$) qui est condensé avec B pour donner F:

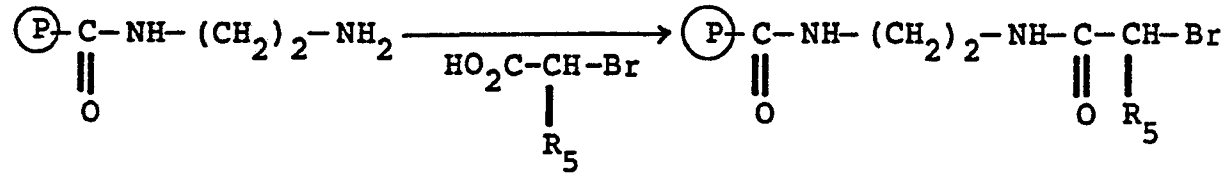

B E F

Ce type d'ancrage, déjà employé sur des résines polystyréniques (MIZOGUCHI, SHIGEZAN et TAKAMURA, Chem. Pharm-Bull., 1970, 18, 1465; WANG, J. Org. Chem, 1976, 41, 3258), est original dans le cas d'un support polyacrylique.

La fixation de l'aminoacide C-terminal dans la première étape, et le clivage final du peptide de son support sont réalisés dans les mêmes conditions que ci-dessus.

Les résines ainsi fonctionnalisées et utilisées en synthèse peptidique ont un taux de fonctionnalisation compris eentre 0,2 et 5 mèq par gramme et, de préference. entre 1,0 et 1,2 mèq par gramme de support sec.

La fixation sur la résine de l'aminoacide C-terminal, dont le $NH_2$ est protégé par exemple par le groupe acido-labile t-butyloxycarbonyle (Boc) ou par le groupe baso-labile fluorénylméthyloxycarbonyle (Fmoc) peut être réalisée par plusieurs voies:

- par l'intermédiaire du sel de césium pour X = Cl ou Br,
- par action du dicyclohexylcarbodiimide (DCC) en présence de diméthylamino-4 pyridine (DMAP) comme catalyseur, pour X = OH,
- ou encore, dans le cas où X = OH, en préparant extemporanément l'anhydride symétrique de l'aminoacide N-protégé, et en le faisant réagir sur D en présence de DMAP.

Le clivage final du peptide de son support est réalisé:

- par une base minérale en solution diluée lorsque Y est un groupe méthylène ou lorsqu'on utilise F; avec $NH_3/CH_3OH$ il est poss ible d' obtenir un amide-C terminal;
- par un acide organique tel que l'acide trifluoracétique en solution diluée, lorsque Y représente un groupe $-CH_2-O$

Méthodologie de la synthèse peptidique

Les supports faisant l'objet de l'invention ont la propriété de présenter une bonne perméabilité et de gonfler notablement dans une large gamme de solvants protiques et aprotiques, ainsi que dans des tampons aqueux de pH variés.

Dans ces conditions, lors d'un couplage, la résine porteur du N-Boc ou du N-Fmoc aminoacide C-terminal (ou du peptide en cours d'élaboration et convenablement protégé) se comporte corrme une "pseudo-solution" et le gel ainsi constitué est infiltrable. Ceci crée des conditions particulièrement favorables, se rapprochant de celles de la phase homogène, pour la formation de la liaison peptidique. La réaction terminée, l'addition d'un tiers solvant (éther, THF, dioxanne) provoque l'expulsion du solvant perméant la résine et la précipitation de celle-ci sous une forme aisément et rapidement filtrable, contrairement à ce qui se passe dans l'état de la technique.

Ces propriétés particulières et spécifiques des résines de l'invention conduisent à une augmentation significative de la vitesse (multipliée au moins par trois, par rapport à ce qui est connu) et du rendement des couplages. Il en résulte, pour chaque couplage individuel, la possibilité de diminuer notablement la valeur du

rapport: nombre de moles d'aminoacide N-protégé/taux de fonctionnalisation du support: 1,1 à 1,2 alors que les valeurs usuelles sont de l'ordre de 2,5. En outre, il n'y a jamais de colmatage des colonnes lors des filtrations intermédiaires.

L'alternance du couplage en phase quasi-homogène et de la précipitation du support constitue ainsi une méthodologie nouvelle, simplifiée et améliorée de synthèse peptidique en phase solide.

Le protocole d'une opération de couplage peut, à titre d'exemple, être le suivant:

1. Addition d'un solvant inerte tel que $CHCl_3$, $CH_2Cl_2$ ou DMF pour gonfler la résine supportant le N-Boc aminoacide C-terminal (ou le peptide convenablement protégé);
2. Un lavage à l'aide d'un éther linéaire ou cyclique tel que l'éther éthylique, l'éther isopropylique, le THF ou le dioxane;
3. Addition d'acide trifluoracétique dans le solvant pour effectuer la déprotection du $NH_2$;
4. Précipitation de la résine par addition d'éther comme en 2;
5. Filtration suivie de quatre lavages à l'éther comme en 2;
6. Neutralisation par addition de diisopropyléthylamine dans un solvant inerte;
7. Quatre lavages à l'éther comme en 2, puis séchage dans un courant d'azote;
8. Couplage par agitation avec une solution, dans le solvant chloré, de l'anhydride symétrique (préparé extemporanément) d'un N-Boc aminoacide;
9. Précipitation par addition d'éther comme en 2 et filtration, et
10. Quatre lavages à l'éther comme en 2.

Cette nouvelle méthodologie a été testée avec succès dans la synthèse de peptides présentant un intérêt biologique par exemple un undécapeptide Ac-Gly-Gly-Lys-Gly-Gly-Ala-Arg-Lys-Val-Leu-Homoser.

**Exemple**

Synthèse du peptide Ac-Gly-Gly-Lys-Gly-Gly-Ala-Arg-Lys-Val-Leu-Homoser.

Le peptide est synthétisé sur une résine acrylique obtenue en copolymérisant la N-acrylylpyrrolidine, l'éthylènebisacrylamide et le dérivé N-acrylique de l'ester méthylique de l'acide ε -aminocaproïque. Le support est ensuite fonctionnalisé de la façon suivante: à 1 g de résine (portant des groupes fonctionnels $CO_2CH_3$), on ajoute 32 ml d'éthylènediamine redistillée et agite pendant une nuit à température ambiante. On filtre, lave à l'eau déminéralisée jusqu'à neutralité, puis à l'éthanol et à l'éther. La résine est finalement séchée pendant 24 h à l'étuve sous vide; elle contient alors 1 meq de $NH_2$ par gramme de polymère sec.

Il a été utilisé 2 g de résine, soit 2 meq en $NH_2$. Le cycle d'opération nécessaire pour accrocher un aminoacide sur la résine est le suivant:

| Solvant ou réactif | N° | Réaction | Volume (ml) | Temps (min). |
|---|---|---|---|---|
| éther éthylique anhydre | 1 | rinçage | 75 | 2 |
| | 2 | | | |
| | 3 | | | |
| | 4 | | | |
| Acide trifluoroacétique 30/ $CH_2Cl_2$70 (V/V) | 5 | Déprotection | 50 | 2 |
| | 6 | | 50 | 30 |
| Ether éthylique anhydre | 7 | Rinçage | 75 | 2 |
| | 8 | | | |
| | 9 | | | |
| | 10 | | | |
| Disopropyléthylamine 10/ $CH_2Cl_2$90 (V/V) | 11 | Neutralisation | 50 | 2 |
| | 12 | | | 30 |
| Ether éthylique anhydre | 13 | Rinçage | 75 | 2 |
| | 14 | | | |
| | 15 | | | |
| | 16 | | | |
| Ajout de l'anhydride symétrique de l'amino acide à coupler dans 50 ml de $CH_2Cl_2$ | 17 | Couplage | 50 | 60 |
| Ether éthylique anhydre | 18 | Rinçage | 75 | 2 |
| | 19 | | | |
| | 20 | | | |
| | 21 | | | |
| Disopropyléthylamine 10/ $CH_2Cl_2$ 90 (V/V) | 22 | Neutralisation | 50 | 2 |
| | 23 | | | 30 |
| Ether éthylique anhydre | 24 | Rinçage | 75 | 2 |
| | 25 | | | |
| | 26 | | | |
| | 27 | | | |
| Ajout de l'anhydride symétrique de l'amino acide à coupler dans 50 ml de $CH_2Cl_2$ | 28 | Couplage | 50 | 30 |
| Ether éthylique | 29 | Rinçage | 75 | 2 |
| | 30 | | | |
| | 31 | | | |
| | 32 | | | |

Vérification du déroulement du couplage à l'aide du test de Kaiser à la ninhydrine.

Comme le décrit le processus expérimental, il a été systématiquement effectué deux couplages pour chaque acide aminé introduit. La réaction de couplage est effectuée à l'aide des anhydrides symétriques qui sont préparés de la manière suivante:

1 mmol de l'acide aminé protégé est dissoute dans 20 ml de $CH_2Cl_2$ anhydre, la solution est refroidie à 0° et on ajoute alors 0,5 mmole de dicyclohexylcarbodiimide en solution dans 10 ml de $CH_2Cl_2$. Après 20 minutes de réaction à 0°C, on filtre le précipité de dicyclohexylurée qui est rincé avec 20 ml de $CH_2Cl_2$, les solutions sont jointes et mises en contact avec la résine dans le réacteur.

Par cette méthode, ont pu être préparés les anhydrides symétriques à partir de Boc Gly OH, $N^\varepsilon$ Z $N^\alpha$ Boc Lys OH Boc Val OH, Boc Leu OH, Boc Met OH, et Ac Gly OH. Dans le cas de $N^G$ $NO_2$ $N^\alpha$ Boc Arg OH, le produit est dissous dans le minimum de DMF et le volume complété à 20 ml avec du chlorure de méthylène sec; ensuite la réaction est effectuée comme il est décrit plus haut.

Après couplage du dernier aminoacide la résine est séchée sous vide poussé en présence de $P_2O_5$ durant 24 heures puis mise en suspension dans 50 ml d'acide trifluoroacétique. On fait alors barboter durant 2 heures un lent courant de HBr sec. On filtre et on rince abondarrment avec $CH_2Cl_2$ et $Et_2O$. Cette acidolyse déprotège les chaînes latérales des lysines.

La poudre rouge orangée obtenue est alors mise en suspension dans 100 ml d'acide trifluoroacétique 2 M et

on aloute 5 g de zinc en poudre. Après 12 heures d'agitation à température ambiante, on lave abondamment avec HCl 2N, à l'eau, à l'éthanol et finalement à l'éther éthylique anhydre. Cette hydrogénolyse clive le nitro porté par le guanidino de l'arginine.

Après séchage de la résine sous vide poussé et sur $P_2O_5$ durant 12 heures, on prélève un grarrme de polymère qui est traité, par 2,5 g de bromure de cyanogène dans 70 ml d' acide propionique. Après 24 heures de réaction à température ambiante, on lyophylise et on obtient le peptide désiré avec un rendement de 45 %.

**Revendications**

pour les états contractants BE CH DE FR GB IT LI LU NL SE

1. Nouvel les résines fonctionnalisées à l'éthylène diamine, la fonctionnalisation consistant en une amidification de la fonction ester

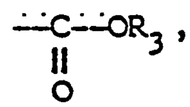

dérivant des résines obtenues par copolymérisation des trois monomères distincts définis comme suit:

I - Un monomère défini comme étant la maille du support et qui consiste en une N-acrylylpolyméthylèneimine de formule:

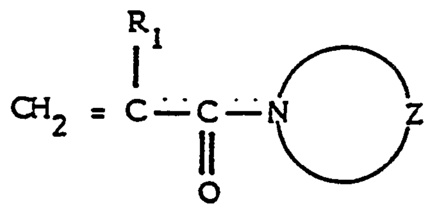

I

avec $R_1 = H$ ou $-CH_3$

$$Z = -(CH_2)_{n_1}- \quad \text{avec } n_1 = 4, 5 \text{ ou } 6$$

ou

$$-(CH_2)_2-X-(CH_2)_2- \quad \text{avec } X = O \text{ ou } N-CH_3$$

ou un N-acrylyldialcoylamide, de formule:

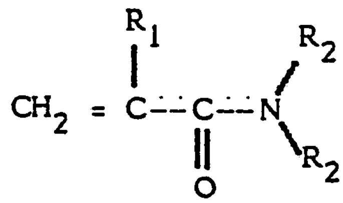 2

avec $R_1$ ayant la signification précédente

$R_2 = -CH_3$ ou $-C_2H_5$

II - Un monomère défini comme étant l'agent de réticulation et qui consiste en un N,N' -bisacrylyldiaminoalcane de formule:

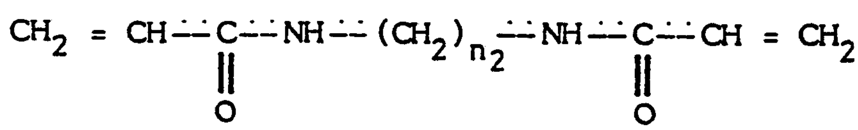 3

avec $n_2 = 1$ ou $2$

III - Par gramme de support sec I, 0,2 à 5 mmol/gr d'un monomère défini conme étant l'agent de fonctionnalisation et qui consiste en un N-acrylylaminoacide ou ester, de formule:

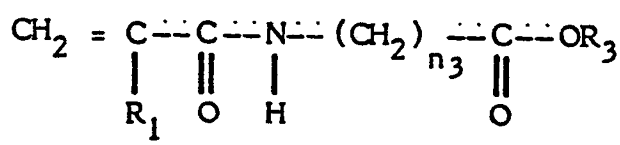 4

avec $R_1$ et $R_3$ = H ou $-CH_3$

$n_3 = 1, 2, 3$ ou $5$

ou un N-acrylylaminoacide (ou ester) asymétrique de serie L, de formule:

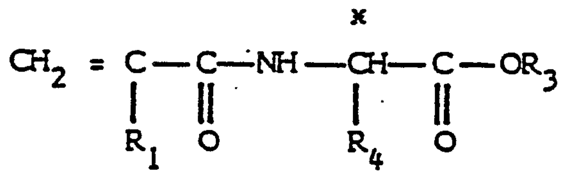

5

avec $R_1$ et $R_3$ = H ou $-CH_3$

$R_4$ = $-CH_3$

$-CH(CH_3)_2$

$-CH_2-CH(CH_3)_2$

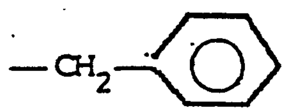

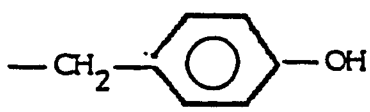

$-CH_2-CH_2-S-CH_3$

$-(CH_2)_4-NH_2$

ou encore la N-acrylyl (L) proline, ou son ester méthylique, de formule:

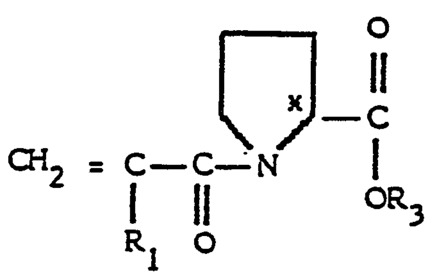

6

avec $R_1$ et $R_3$ = H ou $-CH_3$

2. Procédé de préparation des résines selon la revendication 1, caractérisé en ce que le traitement à l'éthylène diamine conduisant à l'amidification de la fonction ester

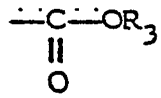

du monomère III, s'effectue entre 0° et 25° C.

3. Procédé selon la revendication 2, caractérisé en ce que la réaction s'effectue en présence de dicyclohexylcarbodiimide lorsque $R_3$ = H.

4. Procédé selon la revendication 2, caractérisé en ce que la réaction s'effectue en présence d'un excès d'éthylène diamine anhydre lorsque $R_3$ = $CH_3$.

5. Résines selon la revendication 1, caractérisées en ce qu'elles comportent, fixé au radical aminé libre de l'éthylène diamine, un système d'ancrage réversible constitué par le reste

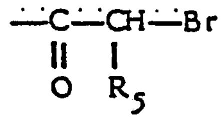 ,

$R_5$ représentant H ou $CH_3$.

**Revendication**

pour l'état contractant AT

1. Procédé de préparation de nouvelles résines fonctionnalisées à l'éthylène diamine, caractérisées en ce qu'on fait réagir, entre 0° et 25° C, l'éthylène diamine sur une résine obtenue par copolymérisation des trois monomères distincts définis comme suit:

I - Un monorrère défini comme étant la maille du support et qui consiste en une N-acrylylpolyméthylèneimine de formule:

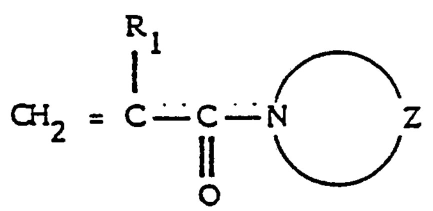

I

avec $R_1$ = H ou $-CH_3$

$Z = -(CH_2)_{n_1}-$ avec $n_1$ = 4, 5 ou 6

ou

$-(CH_2)_2-X-(CH_2)_2-$ avec X = O ou 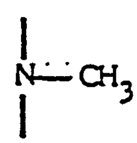

ou un N-acrylyldialcoylamide, de formule:

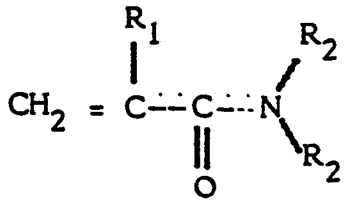

2

avec $R_1$ ayant la signification précédente

$R_2 = -CH_3$ ou $-C_2H_5$

II - Un monomère défini comme étant l'agent de réticulation et qui consiste en un N,N' -bisacrylyldiaminoalcane de formule:

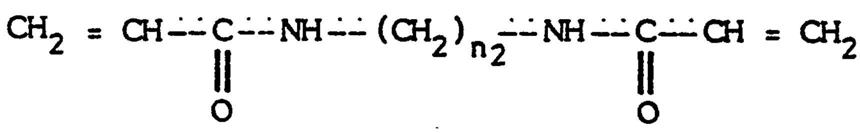

3

avec $n_2 = 1$ ou 2

III - Par gramme de support sec 1, 0,2 à 5 mmol d'un monomère défini comme étant l'agent de fonctionnalisation et qui consiste en un N-acrylylaminoacide ou ester, de formule:

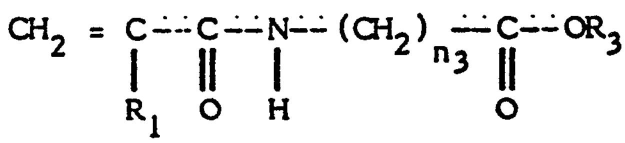

4

avec $R_1$ et $R_3$ = H ou $-CH_3$

$n_3 = 1, 2, 3$ ou 5

ou un N-acrylylaminoacide (ou ester) asymétrique de série L, de formule:

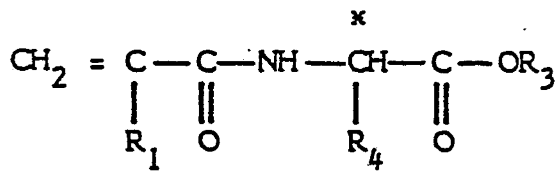

5

avec $R_1$ et $R_3$ = H ou $-CH_3$

$R_4$ = $-CH_3$

$-CH(CH_3)_2$

$-CH_2-CH(CH_3)_2$

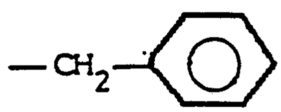

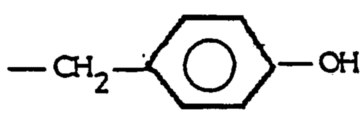

$-CH_2-CH_2-S-CH_3$

$-(CH_2)_4-NH_2$

ou encore la N-acrylyl (L) proline, ou son ester méthylique, de formule:

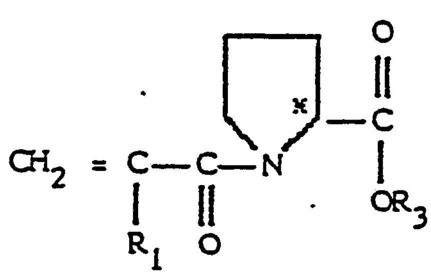

6

avec $R_1$ et $R_3$ = H ou $-CH_3$

2. Procédé selon la revendication 1, caractérisé en ce que la réaction s'effectue en présence de dicyclohexylcarbodiimide lorsque $R_3$ = H.

3. Procédé selon la revendication 1, caractérisé en ce que la réaction s' effectue en présence d'un excès d'éthylène diamine anhydre lorsque $R_3 = CH_3$.

**Patentansprüche**

für die Vertragsstaaten BE CH DE FR GB IT LI LU NL SE

1. Neue äthylendiaminfunktionelle Harze, bei denen die Funktionalisierung aus einer Amidisierung der funktionellen Estergruppe

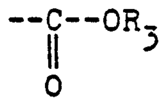

besteht, abgeleitet von
Harzen, die durch Copolymerisation der drei folgendermaßen definierten Monomeren entstehen:
I - Ein Monomeres, welches als Stützzelle dient und aus einem N-Acrylylpolymethylenimin der Formel:

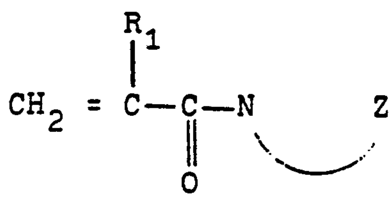

1

mit $R_1$ = H oder $-CH_3$

$Z = -(CH_2)_{n_1}-$ mit $n_1$ = 4, 5 oder 6

oder

$-(CH_2)_2-X-(CH_2)_2-$ mit X = O oder 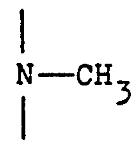

oder einem N-Acrylyldialcolylamid der Formel:

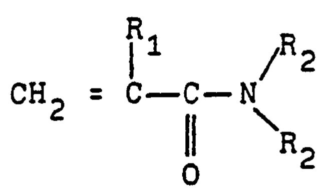

2

wobei $R_1$ die oben angegebene Bedeutung hat und

$R_2 = -CH_3$ oder $-C_2H_5$

ist und
II - Einen Monomer welches als Vernetzungsmittel wirkt und aus einem N,N'-Bisacryiyldiaminoalkan der Formel:

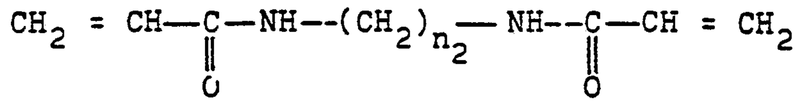 3

mit $n_2$ = 1 oder 2 und

III - Pro Gramm Trockensubstanz des Monomeres I, 0,2 bis 5 mmol eines Monomeren das als Funktionisierungsagens dient und dass aus einem N-Acrylylaminoacid oder Ester der Formel:

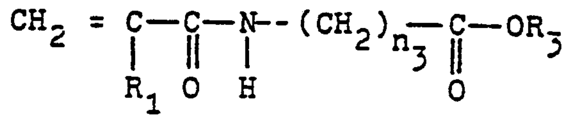 4

mit $R_1$ und $R_3$ = H oder $-CH_3$

$n_3$ = 1, 2, 3 oder 5

oder einem asymmetrischen N-Acrylylaminoacid (oder Ester) der Serie L der Formel:

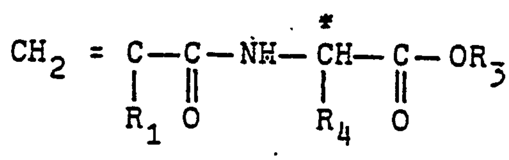 5

mit $R_1$ und $R_3$ = H oder $-CH_3$

$R_4$ = $-CH_3$

$-CH(CH_3)_2$

$-CH_2-CH(CH_3)_2$

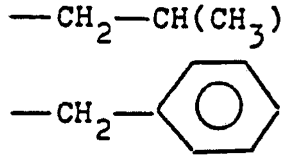

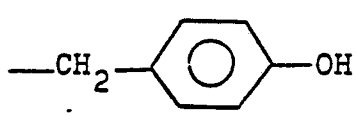

$-CH_2-CH_2-S-CH_3$

$-(CH_2)_4-NH_2$

oder auch einem N-acrylyl (L) Prolin oder dessen Methylester der Formel:

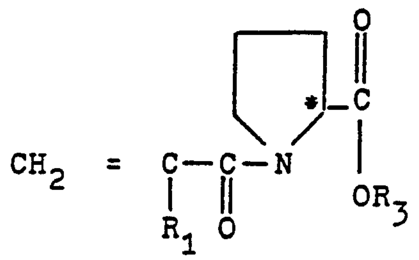

6

mit $R_1$ und $R_3$ = H oder $-CH_3$

2. Verfahren zur Herstellung der Harze nach Anspruch 1, dadurch gekennzeichnet, daß die Behandlung mit Äthylendiamin, die zur Amidifizierung der funktionalen Estergruppe

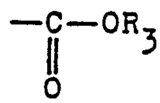

des Monomeren III führt, zwischen 0° und 25°C durchgeführt wird.

3. Verfahren nach Anspruch 2, dadurch gekennzeichnet, daß die Reaktion in Gegenwart von Dicyclohexylcarbodiimid durchgeführt wird, wenn $R_3$ = H.

4. Verfahren nach Anspruch 2, dadurch gekennzeichnet, daß die Reaktion in Gegenwart eines Überschusses von Äthylendiaminanhydrid durchgeführt wird, wenn $R_3 = CH_3$.

5. Harze nach Anspruch 1, dadurch gekennzeichnet, daß sie am freien Aminradikal des Äthylendiamins reversibel getunden ein durch den Rest ge-

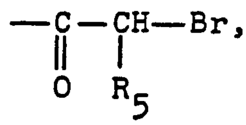

bildetes Verankerungssystem, wobei $R_5$ H oder $CH_3$, bedeutet, aufweisen.

**Patentansprüche**

für den Vertragsstaat AT

1. Verfahren zur Herstellung neuer Äthylendiamin funktionalisierter Harze, dadurch gekennzeichnet, daß man zwischen 0° und 25°C Äthylendiamin mit einem Harz reagieren läßt, welches durch Copolymerisation der drei im folgenden definierten Monomere erhalten wird:

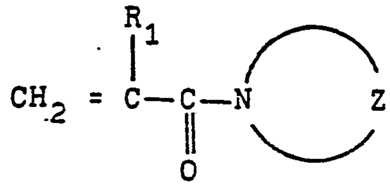 1

mit $R_1$ = H oder $-CH_3$

$Z = -(CH_2)_{n_1}-$ mit $n_1$ = 4, 5 oder 6

oder

$-(CH_2)_2-X-(CH_2)_2-$ mit X = O oder 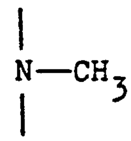

oder einem N-Acrylyldialcolylamid der Formel:

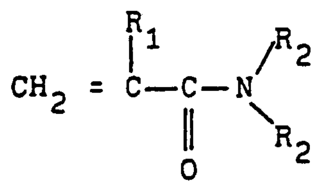 2

wobei $R_1$ die oben angegebene Bedeutung hat und

$R_2 = -CH_3$ oder $-C_2H_5$

ist und

II - Einen Monomer, welches ais Vernetzungsmittel wirkt und aus einem N,N'-Bisacrylyldiaminoalkan der Formel:

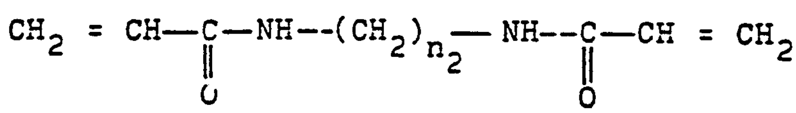 3

mit $n_2$ = 1 oder 2 und

III - Pro Gramm Trockensubstanz des Monomeres I, 0,2 bis 5 mmol eines Monomeren das als Funktionisierungsagens dient und dass aus einem N-Acrylylaminoacid oder Ester der Formel:

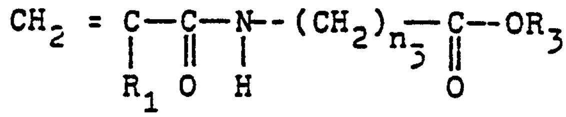 4

mit $R_1$ und $R_3$ = H oder $-CH_3$

$n_3$ = 1, 2, 3 oder 5

oder einem asymmetrischen N-Acrylylaminoacid (oder Ester) der Serie L der Formel:

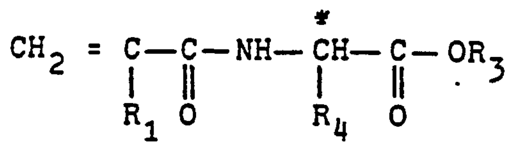 5

mit $R_1$ und $R_3$ = H oder $-CH_3$

$R_4 = -CH_3$

$-CH(CH_3)_2$

$-CH_2-CH(CH_3)_2$

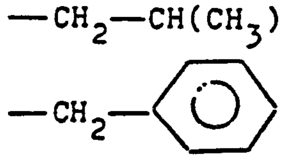

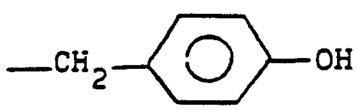

$-CH_2-CH_2-S-CH_3$

$-(CH_2)_4-NH_2$

oder auch einem N-Acrylyl (L) Prolin oder dessen Methylester der Formel:

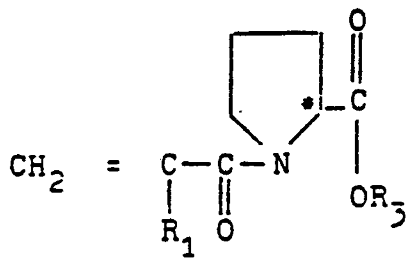

6

mit $R_1$ und $R_3$ = H oder $-CH_3$

2. Verfahren nach Anspruch 1, dadurch gekennzeichnet, daß die Reaktion in Gegenwart von Dicyclohexylcarbodiimid durchgeführt wird, wenn $R_3 = H$.

3. Verfahren nach Anspruch 1, dadurch gekennzeichnet, daß die Reaktion in Gegenwart eines Überschusses von Äthylendiaminanhydrid durchgeführt wird, wenn $R_3 = CH_3$.

**Claims**

for the contracting states BE CH DE FR GB IT LI LU NL SE

1. New functionalised resins of ethylene diamine, the functionalisation consisting of an amidification of the ester function

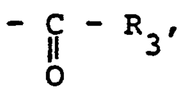

deriving from resins obtained by copolymersiation of three distinct monomers defined as follows:

1 - a monomer defined as being the support matrix and which consists of an N-acrylylpolymethyleneimine of formula:

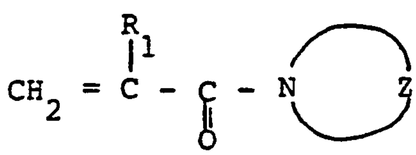

1

with $R_1$ = H or $-CH_3$

$Z = -(CH_2)_{n_1}-$ with $n_1$ = 4,5 or 6

or

$-(CH_2)_2 - X - (CH_2)_2-$ with X = O or N $-CH_3$

or an N-acrylyldialkylamide, of formula:

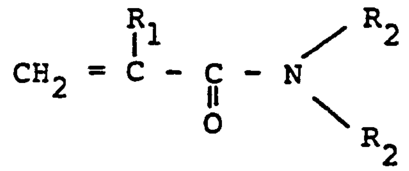

2

with $R_1$ having the aforesaid meaning

$R_2 = -CH_3$ or $-C_2H_5$

II - a monomer defined as being the cross-linking agent and which consists of an N,N'-bisacrylyldiaminoalkane of formula:

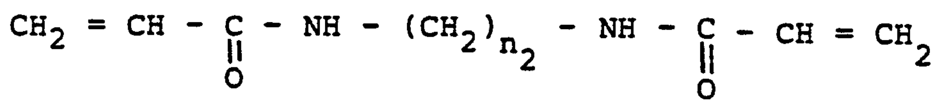

3

with $n_2 = 1$ or $2$

III - per gram of dry support 1, 0.2 to 5 mmol of a monomer defined as being the functionalisation agent and which consists of an N-acrylylaminoacid or ester, of formula:

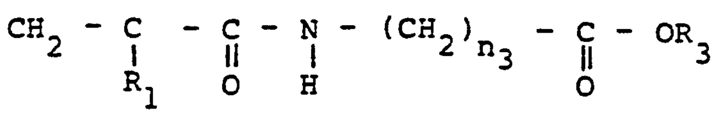

4

with $R_1$ and $R_3$ = H or $-CH_3$

$n_3 = 1, 2, 3$ or $5$

or an asymetric N-acrylylaminoacid (or ester) of L series, of formula:

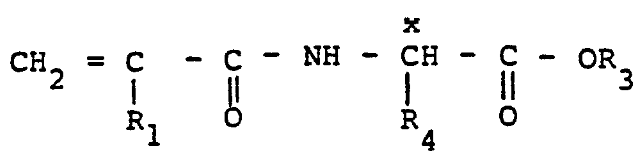

5

with $R_1$ and $R_3$ = H or $-CH_3$

$R_4 =$ $-CH_3$

$-CH(CH_3)_2$

$-CH_2 - CH(CH_3)_2$

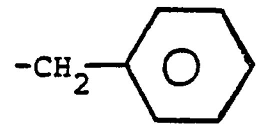

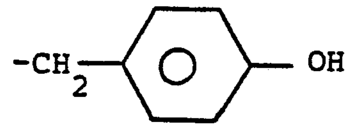

$-CH_2- CH_2 - S - CH_3$

$-(CH_2)_4 - NH_2$

or finally N-acrylyl (L) proline, or its methyl ester, of formula:

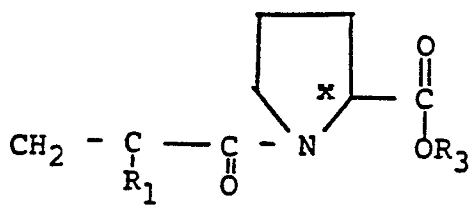

with $R_1$ and $R_3$ = H or $-CH_3$

2. Process of preparation of resins according to claim 1, characterised in that the treatment of the ethylene diamine leading to the amidification of the ester function

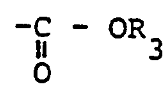

of the monomer III is effected between 0° and 25°C.

3. Process according to claim 2, characterised in that the reaction is effected in the presence of dicyclohexylcarbodiimide when $R_3$ = H.

4. Frocess according to claim 2, characterised in that the reaction is effected in the presence of an excess of anhydrous ethylene diamine when $R_3$ = CH.

5. Resins according to claim I, characterised in that they include, attached to the free amine radical of the ethylene diamine, a reversible anchorage system constituted by the residue

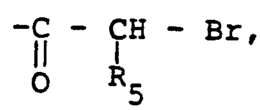

, $R_5$

representing H or $CH_3$.

**Claims**

for the contracting state AT

1. Process of preparation of new functionalised resins of ethylene diamine, characterised in that ethylene diamine is reacted, between 0° and 25°C, with a resin obtained by copolymerisation of three distinct monomers defined as follows:

I-a monomer defined as being the support matrix and which consists of an N-acrylylpolymethyleneimine of formula:

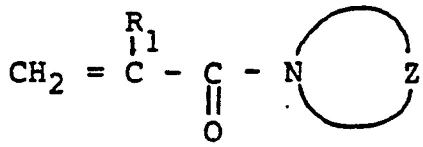

1

with $R_1$ = H or $-CH_3$

$X = -(CH_2)_{n_1}-$ with $n_1$ = 4,5, or 6

or

$-(CH_2)_2-X-(CH_2)_2-$ with X = O or $\geq N-CH_3$

or an N-acrylyldialkylamide, of formula:

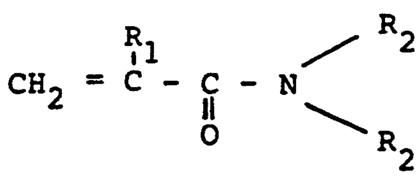

2

with $R_1$ having the aforesaid meaning

$R_2 = -CH_3$ or $-C_2H_5$

II - a monomer defined as being the cross-linking agent and which consists of an N, N'-bisacrylyldiaminoalkane of formula:

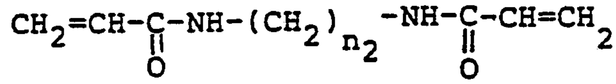 3

with $n_2$ = 1 or 2

III - per gram of dry support I, 0.2 to 5 mmol of a monomer defined as being the functionalisation agent and which consists of an N-acrylylaminoacid or ester, of formula:

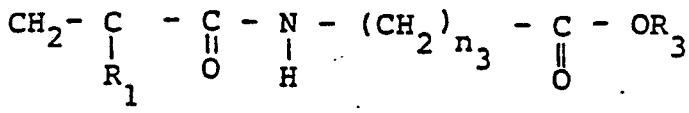 4

with $R_1$ and $R_3$ = H or $-CH_3$

$n_3$ = 1, 2, 3 or 5

or an asymetric N-acrylylaminoacid (or ester) of L series, of formula

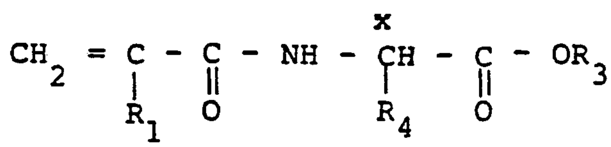 5

with $R_1$ and $R_3$ = H or $-CH_3$

$R_4$ = $-CH_3$

$-CH(CH_3)_2$

$-CH_2-CH(CH_3)_2$

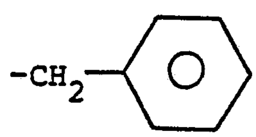

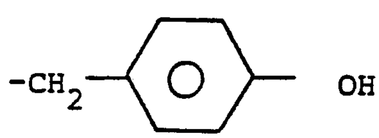

$-CH_2-CH_2-S-CH_3$

$-(CH_2)_4-NH_2$

or finally N-acrylyl (L) proline, or its methyl ester, of formula:

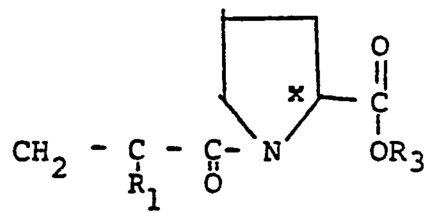

with $R_1$ and $R_3$ = H or $-CH_3$

2. Process according to claim 1 characterised in that the reaction is effected in the presence of dicyclohexylcarbodiimide when $R_3 = H$.

3. Process according to claim 1 characterised in that the reaction is effected in the presence of an excess of anhydrous ethylene diamine when $R_3 = CH_3$.